# EUROPEAN PATENT SPECIFICATION

(11) **EP 3 265 070 B1**
(45) Date of publication and mention of the grant of the patent: **03.07.2019**
(21) Application number: 16708177.7
(22) Date of filing: 07.03.2016
(51) Int. Cl.: A61K 31/122, A61K 31/167, A61K 31/355, A61K 31/375, A61K 31/385, A61K 31/4045, A61P 1/00, A61P 15/02, A61P 39/06

(54) **MELATONIN FOR PREVENTING AND TREATING RADIATION VAGINITIS AND PROCTITIS**
MELATONIN ZUR VORBEUGUNG UND BEHANDLUNG VON STRAHLUNGSVAGINITIS UND STRAHLUNGSPROKTITIS
MÉLATONINE POUR LA PRÉVENTION ET LE TRAITEMENT DE LA VAGINITE RADIQUE ET DE LA PROCTITE RADIQUE

(30) Priority: 06.03.2015 DK 201570128
(43) Date of publication of application: 10.01.2018
(73) Proprietor: Repoceuticals ApS, 2970 Hørsholm (DK)
(72) Inventor: UTTENTHAL, Lars Otto, 37002 Salamanca (ES)
(74) Representative: Zacco Denmark A/S
(86) International application number: PCT/EP2016/054795
(87) International publication number: WO 2016/142341

(56) References cited:
- EP-A1- 2 702 992
- WO-A1-01/07064
- WO-A1-92/11855
- WO-A2-02/41837
- WO-A2-2005/062992
- US-B1- 7 250 445
- DEEPA S MAHARAJ ET AL: "Melatonin: New Places in Therapy", BIOSCIENCE REPORTS, KLUWER ACADEMIC PUBLISHERS-PLENUM PUBLISHERS, NE, vol. 27, no. 6, 7 September 2007 (2007-09-07), pages 299-320, XP019548885, ISSN: 1573-4935, DOI: 10.1007/S10540-007-9052-1
- HUSSEIN M R ET AL: "Melatonin and roentgen irradiation-induced acute radiation enteritis in Albino rats: An animal model", CELL BIOLOGY INTERNATIONAL, ACADEMIC PRESS, GB, vol. 32, no. 11, 1 November 2008 (2008-11-01), pages 1353-1361, XP025574239, ISSN: 1065-6995, DOI: 10.1016/J.CELLBI.2008.08.001 [retrieved on 2008-08-13]

## Description

### Field of invention

The present invention provides compositions comprising melatonin or a derivative thereof as the essential ingredient for preventing and treating vaginal injury due to ionizing radiation by the topical application of the composition to the vaginal mucosa, as defined in the claims. As such, it is particularly relevant to the fields of gynecological oncology and radiotherapy, as well as surgery when this is part of a combined treatment with radiotherapy.

### Background of the invention

### Vaginal radiation injury (radiation vaginitis)

Radiation injury to the vagina commences immediately on exposure to ionizing radiation. Free radicals are produced in the cells which exceed the cells' intrinsic scavenging capabilities, affecting in particular cells of higher mitotic turnover rate, such as the basal cells of the vaginal epithelium. The reduced proliferation of these cells causes an imbalance between production and loss of epithelial cells, resulting in partial or, in the worst cases, complete vaginal denudation. The early stage can be termed "acute radiation vaginitis". Edema and inflammation of the epithelium and connective tissues follow and the small blood vessels show endothelial damage and micro-thrombi. The subsequent reduced blood supply, tissue hypoxia, loss of elastin, collagen deposition and hyalinization and fibrosis leads to thinning of the vaginal mucosa, loss of lubrication, scarring and fibrosis. This causes a shorter, less elastic and dryer vagina. The fibrosis results in varying degrees of narrowing, termed vaginal stenosis, and shortening, simply termed "short vagina". Some women experience complete loss of a functioning vagina. Additionally, estrogen deficiency resulting from radiation-induced menopause or natural menopause, or cessation of prior hormone replacement therapy, may intensify the loss of elasticity and lubrication and thinning and atrophy of the vaginal mucosa (see Miles 2012).

Vaginal radiation injury leading to some degree of vaginal stenosis has been variably estimated to affect between 1.2% and 88% of women subjected to pelvic radiotherapy. Brand et al (2006) found that 38% of women treated for cervical cancer with pelvic and/or vaginal radiotherapy had stenosis (27% partial stenosis or shortening, 11% complete stenosis) at 6-month follow-up. The frequency of stenosis was higher in patients over 50 years old.

Irradiation may take the form of external beam radiotherapy or brachytherapy, applied with an internal vaginal applicator, or both. It may be used alone or after surgery for cervical or endometrial cancer. There is a tendency for vaginal stenosis to occur more frequently with brachytherapy or combined radiotherapy, and when radiotherapy is combined with surgery. The severity of vaginal stenosis appears to be related to a higher dose per fraction of brachytherapy, an increased number of fractions and a smaller diameter of the brachytherapy applicator. It has also been noted that vaginal radiation injury is greater when the entire length of the vagina is irradiated rather than the vaginal apex, and the lower vagina has a poorer tolerance to radiation than the upper vagina.

Current medical treatment of vaginal radiation injury is symptomatic, by means of topical estrogen, topical anti-inflammatory benzydamine and systemic hormones. There is clearly a need for a more effective medical prevention and treatment that is directed at the root cause of the pathology.

### Summary of the invention

The invention is defined by the claims. Any subject matter falling outside the scope of the claims is provided for information purposes only.

The invention consists of providing pharmaceutical compositions comprising melatonin or an antioxidant metabolite, derivative or analogue thereof (individually referred to as the protective agent) for the prevention and treatment of vaginal radiation injury by the direct administration of the compositions to the vaginal epithelium as defined in the claims, preferably in the form of a foam, gel, cream or ointment, or as a medicated pessary or suppository. The advantage of the invention is that the protective agent is delivered at high dose directly to the tissue for which protection from radiation damage is desired, while there is no direct delivery to the tumor that is to be treated by radiotherapy. A further advantage will be that the directly applied melatonin will not be subject to the low bioavailability of melatonin given orally, which is subject to individually variable first-pass metabolism in the liver. The compositions are intended to be administered immediately before each dose of radiotherapy is given, i.e. 15 to 30 minutes before a course of radiotherapy. Pharmaceutical compositions are also provided, but not claimed, which comprise melatonin or an antioxidant metabolite, derivative or analogue thereof and a pharmaceutically acceptable form of vitamin E and/or coenzyme Q10 and/or alpha-lipoic acid and/or vitamin C.

Accordingly, the pharmaceutical compositions comprise essentially:
A composition comprising melatonin or an antioxidant metabolite, derivative or analogue thereof formulated to be suitable for administration to the vaginal epithelium as a foam, gel, cream or ointment, or as a medicated pessary or suppository, for the prevention and treatment of vaginal radiation injury.

A composition according to that described above, comprising additionally a pharmaceutically acceptable form of vitamin E and/or coenzyme Q10 and/or alpha-lipoic acid vitamin C.

The invention fulfills the medical need for a preventive, pre-emptive and continuing treatment of the root intracellular cause of radiation injury to the vagina, for which current treatments are symptomatic or directed at post-hoc alleviation of longer-term pathological consequences.

In the following detailed description of the invention, details of the scope of the invention will be given, together with details of the practical performance of the invention.

### Detailed description of the invention

The present invention provides compositions comprising melatonin or an antioxidant metabolite, derivative or analogue thereof as the principal active substance to be topically applied to the vaginal epithelium for the prevention and treatment of vaginal radiation injury, as defined in the claims. Also provided, but not claimed, are compositions for the same purpose, which additionally comprises a pharmaceutically acceptable form or derivative or analogue of one or more of the substances vitamin E, coenzyme Q10, alpha-lipoic acid and vitamin C.

### Active ingredients

The principal active ingredient of the compositions of the invention is melatonin or an antioxidant metabolite thereof, as defined in the claims.

### Melatonin

Melatonin (*N*-acetyl-5-methoxytryptamine) is a hormone produced by the pineal gland in human beings and other mammals by enzymatic modification of the amino acid tryptophan. Melatonin is involved in maintaining the circadian rhythm of various biological functions, being secreted in hours of darkness and acting on high-affinity melatonin Gi-coupled transmembrane receptors MT1 and MT2, which are widely distributed in many cells and tissues of the body. At the same time melatonin acts at supraphysiological concentrations as a powerful antioxidant and free radical scavenger for ROS and reactive nitrogen species (Gomez-Moreno et al 2010). Melatonin can also activate cytoprotective antioxidative enzymes such as copper-zinc and manganese superoxide dismutases (CuZnSOD and MnSOD) and glutathione peroxidase (Rodriguez et al 2004). Melatonin also has anti-inflammatory effects to prevent the upregulation or cause the down-regulation of the expression of nuclear factor kappa B (NF-κB) and pro-inflammatory cytokines such as tumor necrosis factor alpha (TNF-α) and interleukin 1 beta (IL-1β).

**Melatonin as an agent to protect against radiation injury:** Because of melatonin's efficiency as a free radical scavenger, especially of hydroxyl radicals (Tan et al 1993) and ROS, it has been proposed as an agent to protect against radiation injury to cells and tissues. The protective effect of high dose systemic melatonin against the harmful effects of whole-body irradiation has been studied chiefly in rodents. Melatonin has typically been given at intravenous or intraperitoneal doses of 5 mg to 100 mg per kilogram of body weight and protective effects on DNA and nuclear morphology, as well as prolonged survival after lethal doses of irradiation have been observed. Melatonin has been demonstrated to protect against the adverse effects of all relevant
wavelengths ionizing radiation from ultraviolet through x-rays to gamma rays. The results of such studies have been reviewed by Vijayalaxmi et al (2004). In human beings, a protective effect of prior oral melatonin dosage on the damage caused by subsequent *ex vivo* radiation exposure of lymphocytes has been observed (Vijayalaxmi et al 1996). However, controlled clinical trials of the protective effect of oral or systemic melatonin on radiation damage in patients undergoing radiotherapy are lacking.

The above experiments suggest that a major part of the protective effect of melatonin against radiation damage depends on the intracellular presence of melatonin at the time of radiation. This would be consistent with the near instantaneous intracellular production of free radicals as a result of radiation and their initiation of DNA and mitochondrial damage leading to cell death. There has been some concern that pre-treatment with systemically administered melatonin would also diminish the effectiveness of radiotherapy to kill tumor cells. On the other hand, the effects of melatonin to activate cytoprotective enzymes and down-regulate pro-inflammatory cytokines points to a longer term effect that might contribute to protection against radiation damage.

There are various reports on the use of melatonin as an ingredient in topical skin creams for protection again ultraviolet (UV) irradiation (sun-burn), sometimes in combination with known UV-blocking (sun-screen) agents. Products of this type are available as over-the-counter skin creams. One such agent (PraevoSkin by Praevomed GmbH) has undergone preliminary testing for protection against radiation dermatitis of the chest region in a small number of women undergoing radiotherapy for breast cancer (Ben-David et al 2010). The composition of this preparation, said to be an emulsion containing melatonin, has not been made available. A clinical trial (NCT00840515) was registered in 2009, but has not been reported. This preparation and other over-the-counter melatonin-containing skin preparations have not been proposed for, and are not adapted to vaginal or rectal use.

EP 2 702 992 A1 (Escames & Acuña 2014) discloses the use of melatonin and a series of its derivatives for the treatment or prevention of mucositis. While the emphasis is on a topical melatonin gel to treat oral mucositis, mucositis due to radiation or cytotoxic therapy can affect the whole gastrointestinal tract from mouth to anus, including intestinal mucositis, which is also mentioned as a possible target for treatment by the compositions of the invention. However, no mention is made of the vagina or the treatment of radiation vaginitis.

### Melatonin derivatives, analogues and metabolites

Many chemical derivatives of melatonin, including breakdown products and natural metabolites of melatonin, retain the antioxidant and free-radical scavenging properties of the parent molecule.

This makes melatonin a more effective antioxidant than other natural antioxidants such as vitamins C and E (cited by Reiter et al 2007). However, these vitamins show synergy with melatonin with respect to antioxidant activity. In non-hepatic tissues, the reaction of melatonin with two hydroxyl radicals yields the metabolite cyclic 3-hydroxymelatonin (C3-OHM), which undergoes further oxidation by two hydroxyl radicals to break the indole ring and form *N*¹-acetyl-*N*²-formyl-5-methoxykynuramine (AFMK) (Tan et al 1993; Reiter et al 2007). C3-OHM is therefore also an effective antioxidant and hydroxyl radical scavenger. The reaction of melatonin with the hydroxyl radical precursor, hydrogen peroxide, similarly leads to production of AFMK. AFMK is also a reducing agent, capable of donating electrons to detoxify radical species, and has been shown to preserve the integrity DNA exposed to oxidizing agents. The action of aryl formamidase or catalase on AFMK produces *N*¹-acetyl-5-methoxykynuramine (AMK), which is an even more effective scavenger of hydroxyl radicals and reactive nitrogen species, protecting proteins from oxidative destruction. In this process, 3-acetamidomethyl-6-methoxycinnolinone (AMMC) or 3-nitro-AMK (AMNK) is formed.

The liver is the principal site of the classically reported metabolic pathway for melatonin. This consists chiefly of 6-hydroxylation by the cytochromes P450 CYP1A1, CYP1A2, and CYP1B1, and the formation of the minor metabolite *N*-acetylserotonin by CYP2C19. The main product 6-hydroxymelatonin (6-OHM) is then conjugated at the hydroxyl group to form the 6-OHM glucuronide or 6-OHM sulfate. 6-OHM is an effective free radical scavenger in a variety of situations, but is also reported to show pro-oxidant effects in others. Its status as an antioxidant thus remains equivocal (Maharaj et al 2007).

*N*-acetylserotonin (NAS) is not only the immediate biosynthetic precursor but also a minor metabolite of melatonin. Like 6-OHM, it is conjugated to form the glucuronide or sulfate. Its protective effect against oxidative damage in certain model systems is reportedly 5 to 20 times as strong as that of melatonin (Oxenkrug 2005).

Melatonin can also be chemically modified by introducing chemical groups at one or more of any of its constituent atoms susceptible of such modification or by introducing such groups in de novo synthesis of melatonin analogues or derivatives. Such modifications, which do not alter the fundamental indole structure of melatonin, are herein called derivatives. The fundamental indole structure of melatonin can also be modified by substituting other bicyclic aromatic structures. Such modifications are herein called analogues, which may also have different chemical side groups removed, introduced or modified. Many such analogues and derivatives have been prepared, but most of them have not been tested for their antioxidant or free-radical scavenging properties.

### Natural antioxidants that may act in synergy with melatonin

A large number of natural antioxidant agents that have been used pharmaceutically may potentially act synergically with melatonin. These may have additive antioxidant effects, but only a few have been demonstrated to act synergically. Vitamins C and E have been cited in this context and there is preliminary clinical evidence that their systemic administration is beneficial in radiation proctitis (Kennedy et al 2001). A related but not identical property, which is less well assessed, is their efficiency as free radical scavengers and in conferring protection against the harmful effects of radiation and cytotoxic medication. Further natural antioxidants that come under consideration as conferring addition protective effect are alpha-lipoic acid and coenzyme Q10 (also known as ubidecarenone). Both are effective as free radical scavengers and their capacity to ameliorate radiation damage has been demonstrated *in vitro* and in animal models in which the substances have usually been given intraperitoneally or by dietary supplementation.

**Antioxidant metabolites of melatonin:** Of those described above, *N*¹-acetyl-*N*²-formyl-5-methoxykynuramine (AFMK), 6-hydroxymelatonin (6-OHM) and *N*-acetylserotonin (NAS) can be used in compositions of the invention. Cyclic 3-hydroxymelatonin (C3-OHM) and *N*¹-acetyl-5-methoxykynuramine (AMK) are unstable and hence unsuitable for use in a pharmaceutical composition.

**Antioxidant melatonin derivatives:** The chemical structure of melatonin can be represented as in Figure (I), in which sites suitable for chemical modification by the substitution of different chemical groups have been indicated by R₁, R₂, R₃, R₄, R₅ and R₆. These numbers do not correspond to the conventional numbering of positions in the indole ring of melatonin.

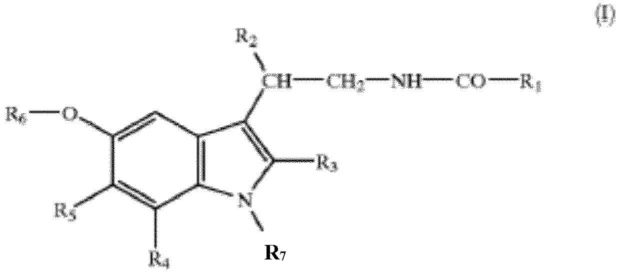

In native melatonin, R₁ and R₆ represent CH₃, while R₂, R₃, R₄, R₅ and R₇ represent H.

Antioxidant melatonin derivatives according to the invention comprise those in which
R₁ represents H, a linear or branched C₁-C₄ alkyl group or a C₁-C₄ alkoxy group,
R₂ represents H or a C₁-C₄ alkyl group,
R₃ represents H, a methyl group or a halogen atom,
R₄ represents H or a halogen atom,
R₅ represents H or a halogen atom,
R₆ represents H or a linear or branched C₁-C₄ alkyl group,
R₇ represents H, a linear or branched C₁-C₄ alkyl group, a -C(=O)-O-Rₐ group or a -C(=O)-N(H)-Rₐ group wherein Rₐ is a linear or branched C₁-C₄ alkyl group,
or pharmaceutically acceptable salts of such derivatives.

**Synergically acting antioxidants:** Described but not claimed are also compositions comprising melatonin or an antioxidant analogue or metabolite thereof together with a synergically acting antioxidant such as vitamin E, coenzyme Q10, alpha-lipoic acid or vitamin C as active substances. Said substances are herein referred to collectively as the active substances or ingredients. In their native forms, vitamin E is an oil, coenzyme Q10 is an almost water-insoluble solid of low melting point, and alpha-lipoic acid is a water-insoluble solid organic acid, while vitamin C is a solid organic acid. These substances must be provided in forms that are appropriate for the pharmaceutical formulation used. For aqueous carriers, a water-soluble form of vitamin E is D-alpha-tocopheryl succinate. Coenzyme Q10 or a suitable antioxidant analogue or derivative thereof, non-limiting examples of which are coenzyme Q9, decylubiquinone and idebenone, may be rendered water-soluble by adsorption to a biologically acceptable carrier such as beta-cyclodextrin during the formulation process. Alpha lipoic acid R-(+)-alpha-lipoic acid, also called (R)-thioctic acid, can be used as its sodium salt, sodium thioctate, which is soluble in water to yield solutions of near-physiological pH. Similarly, a non-limiting example of an appropriate form of vitamin C is sodium ascorbate.

### Formulations

The pharmaceutical composition of the present invention may be in the form of a foam, gel, cream, ointment, emulsion or suspension, which may be delivered by an appropriate applicator or form part of a medicated pessary or suppository.

The formulation typically contains from 1 mg to 100 mg of melatonin or antioxidant metabolite, derivative or analogue thereof per gram of the composition.

In the preferred embodiment, the formulation is a foam suitable for application to the vagina. A non-limiting example of such as formulation is a foam based on propylene glycol, comprising additionally the active ingredients, emulsifying wax, polyoxyl 10 stearyl ether and cetyl alcohol, all of pharmaceutical grade. The foam also contains conservation agents such as methyl and/or propyl parahydroxybenzoate, lactic acid or triethanolamine as a pH regulator, purified water and a suitable propellant, a non-limiting example of which is a hydrocarbon propellant such as HP-70, containing isobutane and propane.

A non-limiting example of an oil-based foam is one comprising pharmaceutical-grade mineral oil, the active ingredients and cetyl or cetyl stearyl alcohol, together with a suitable propellant.

Many other compositions of foam preparations suitable for vaginal use are known to the skilled person and can be applied to the compositions of this invention.

In another embodiment, the formulation is a hydrogel suitable for vaginal application. A non-limiting example of such as formulation is a hydrogel comprising 20% polyethylene and polypropylene copolymers with Pluronic™ F127 poloxamer and a suitable preservative.

In a further embodiment, the formulation is an ointment suitable for vaginal application. A non-limiting example of such as formulation is an ointment comprising liquid paraffin, white vaseline, white wax, hydrogenated castor oil and methyl glucose dioleate.

The normal pH level is reported to be 3.8-4.5 for the vagina. Melatonin and the other active ingredients are all stable at this pH range. Preparations for vaginal use are accordingly adjusted to have a pH in the range of 3.8-4.5.

### Administration

Administration of an effective amount of the pharmaceutical composition is by topical application to the vaginal epithelium by means of an applicator or insert such as a special pessary or suppository.

In the preferred embodiment, the composition is provided in a spray can that also contains a propellant. When the nozzle of the can is depressed, a valve opens to allow the composition-propellant mixture to escape, so that a foam is formed by the expansion of the propellant on emerging. The spray can nozzle is not applied directly to the vagina, but to the nozzle of an applicator in the form of a syringe to receive the foam. The applicator nozzle is smooth and of sufficient length to deliver the foam to upper as well lower regions of the vagina. The foam is applied by hand operation of the applicator syringe to ensure adequate control of the volume and rate of delivery. One gram of composition may expand to about 10 mL of foam.

Gel, cream and ointment preparations are applied with gloved fingers or by means of a smooth applicator. Typically one gram or more, up to 20 grams, of composition may be applied, which has to be dispersed over the whole area of epithelium to be protected.

Compositions which are essentially similar to stiff gels may also be manufactured to coat an appropriately shaped cylindrical pessary or insert made of inert, fibrous material, to place the composition in contact with the epithelium to be protected. The manufacture of such medicated pessaries or inserts are known to those of skill in the art.

### Indications

1. Pelvic external beam radiotherapy for any condition requiring it, including conditions such as cervical carcinoma, endometrial carcinoma.
2. Pelvic brachyradiotherapy for any condition requiring it, including conditions such as cervical carcinoma, endometrial carcinoma.
3. A combination of 1. and 2.

### Dose and dosage regimens

By "effective amount" of the pharmaceutical compositions of the present invention is meant a dose, which, when administered to a subject in need thereof, achieves a concentration which has a beneficial biological effect, i.e. by preventing or reducing radiation injury to the vagina. Such an effective amount may be determined by physicians of ordinary skill in the art attending patients undergoing pelvic radiotherapy and/or brachytherapy.

The effective amounts and dosages of the ingredients of the composition are not determined in relation to body weight or body surface area, because the treatment is topical to the vagina.

The effective amount of melatonin or an analogue, derivative or metabolite thereof for a single dose of topical vaginal administration may be from 1 mg to 100 mg, such as in the range of 1 mg to 50 mg, and especially in the range of 1 mg to 20 mg.

The effective amount of a pharmaceutically acceptable form of vitamin E and/or coenzyme Q10 and/or alpha-lipoic acid and/or vitamin C, in admixture with melatonin or a metabolite, derivative or analogue thereof, may be the same by weight as the amount of melatonin or a metabolite, derivative or analogue thereof.

The effective dose is preferably administered 15 minutes to 30 minutes before each dose of radiation is given. Because melatonin may also have longer term anti-inflammatory effects that are not directly dependent on free radical scavenging, the effective dose may also be given up to six times daily between and after doses of radiation for a period of up to 12 months after the initiation of radiotherapy. The daily dose may be given once a day or in divided or full effective doses two times a day, three times a day, four times a day, five times a day, or six times a day. The total daily dose may thus be from one to six times the amount of a single effective dose.

Duration of dosing will typically range from 3 months to 12 months.

A dose regimen may alternate between periods of administration of the pharmaceutical composition according to the present invention and periods without administration (a pause in treatment). A period with a pause of treatment in such a dose regime may last for 1 week to 2 weeks, or 2 weeks to 3 weeks, or 3 weeks to 1 month, or 1 month to two months, all at the discretion of the attending physician.

### References

Ben-David MA, Elkayam R, Galarenter I, Pfeffer RM (2010) A prospective, double-blind, randomized study of a melatonin-containing cream for radiation-induced breast dermatitis. ASCO 2010 Breast Cancer Symposium, abstract number 123.
Brand AH, Bull CA, Cakir B (2006) Vaginal stenosis in patients treated with radiotherapy for carcinoma of the cervix. Int J Gynecol Cancer 16:288-293.
EP 2 702 992 A1 (Escames G, Acuña D, 2014) Use of melatonin for treating and/or preventing mucositis.
Gomez-Moreno G, Guardia J, Ferrera MJ, Cutando A, Reiter RJ (2010) Melatonin in diseases of the oral cavity. Oral Dis 16:242-247.
Kennedy M, Bruninga K, Mutlu EA, Losurdo J, Choudhary S, Keshavarzian A (2001) Successful and sustained treatment of chronic radiation proctitis with antioxidant vitamins E and C. Am J Gastroenterol 96:1080-1084.
Maharaj DS, Glass BD, Daya S (2007) Melatonin: new places in therapy. Biosci Rep 27:299-320.
Miles T (ed.) (2012) International guidelines on vaginal dilation after radiotherapy; a clinical guideline. Owen Mumford Ltd, Woodstock, UK.
Oxenkrug G (2005) Antioxidant effects of N-acetylserotonin: possible mechanisms and clinical implications. Ann N Y Acad Sci 1053:334-347.
Reiter RJ, Tan DX, Terron MP, Flores LJ, Czarnocki Z (2007) Melatonin and its metabolites: new findings regarding their production and their radical scavenging actions. Acta Biochim Pol 54:1-9.
Rodriguez C, Mayo JC, Sainz RM, Antolín I, Herrera F, Martin V, Reiter RJ (2004) Regulation of antioxidant enzymes: a significant role for melatonin. J Pineal Res 36:1-9.
Tan DX, Chen LD, Poeggeler B, Manchester LC, Reiter RJ (1993) Melatonin: a potent, endogenous hydroxyl radical scavenger. Endocrine J 1:57-60.
Vijayalaxmi, Reiter RJ, Herman TS, Meltz ML (1996) Melatonin and radioprotection from genetic damage: in vivo/in vitro studies with human volunteers. Mutat Res 371:221-228.
Vijayalaxmi, Reiter RJ, Tan DX, Herman TS, Thomas CR Jr (2004) Melatonin as a radioprotective agent: a review. Int J Radiat Oncol Biol Phys 59:639-653.

## Claims

1. A composition comprising melatonin of Formula (I) 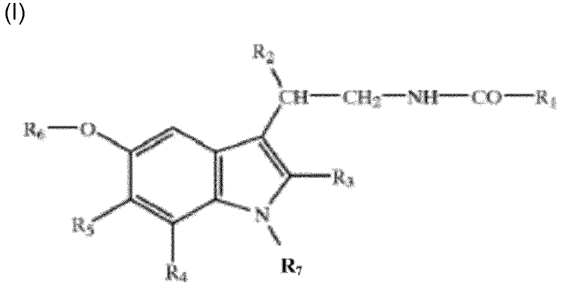 wherein R₁ and R₆ represent CH₃, and R₂, R₃, R₄, R₅ and R₇ represent H, or an antioxidant derivative thereof, wherein
R₁ represents H, a linear or branched C₁-C₄ alkyl group or a C₁-C₄ alkoxy group,
R₂ represents H or a C₁-C₄ alkyl group,
R₃ represents H, a methyl group or a halogen atom,
R₄ represents H or a halogen atom,
R₅ represents H or a halogen atom,
R₆ represents H or a linear or branched C₁-C₄ alkyl group,
R₇ represents H, a linear or branched C₁-C₄ alkyl group, a -C(=O)-O-Rₐ group or a -C(=O)-N(H)-Rₐ group, wherein Rₐ is a linear or branched C₁-C₄ alkyl group,
or antioxidant metabolites thereof of the formula *N*¹-acetyl-*N*²-formyl-5-methoxykynuramine, 6-hydroxymelatonin and *N*-acetylserotonin, for use in the prevention and treatment of vaginal injury due to pelvic external beam radiotherapy and/or due to pelvic brachyradiotherapy, wherein the composition is administered topically to the vaginal epithelium 15 minutes to 30 minutes before an episode of pelvic external beam radiotherapy and/or pelvic brachyradiotherapy.

2. The composition for use according to claim 1 wherein the composition is a foam, gel, cream, ointment or medicated pessary or suppository.

3. The composition for use according to any one of the preceding claims, wherein the pH of the composition is in the range 3.8-4.5.

4. The composition for use according to any one of the preceding claims, wherein the composition is for administration 1, 2, 3, 4, 5, or 6 times per day.

5. The composition for use according to any one of the preceding claims, wherein the composition is for administration over a period of 3 months or more, such as 4 months or more, such as 5 months or more, such as 6 months or more, such as 12 months.

6. The composition for use according to any one of the preceding claims, wherein the composition is in the single standard adult dose of melatonin of 1 mg to 100 mg.

7. The composition for use according to any one of the preceding claims, wherein the daily dose of composition is from one to six times the single doses of claim 6.

## Patentansprüche

1. Zusammensetzung umfassend Melatonin der Formel (I) 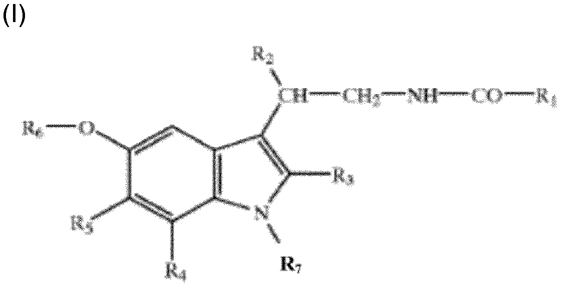
wobei R₁ und R₆ CH₃ repräsentieren, und R₂, R₃, R₄, R₅ und R₇ H oder ein Antioxidansderivat davon repräsentieren, wobei
R₁ H, eine lineare oder verzweigte C₁-C₄-Alkylgruppe oder eine C₁-C₄-Alkoxygruppe repräsentiert,
R₂ H oder eine C₁-C₄-Alkylgruppe repräsentiert,
R₃ H, eine Methylgruppe oder ein Halogenatom repräsentiert,
R₄ H oder ein Halogenatom repräsentiert,
R₅ H oder ein Halogenatom repräsentiert,
R₆ H oder eine lineare oder verzweigte C₁-C₄-Alkylgruppe repräsentiert,
R₇ H, eine lineare oder verzweigte C₁-C₄-Alkylgruppe, eine -C(=O)-O-Rₐ-Gruppe oder eine -C(=O)-N(H)-Rₐ-Gruppe repräsentiert, wobei Rₐ eine lineare oder verzweigte C₁-C₄-Alkylgruppe ist,
oder Antioxidansmetaboliten davon der Formel *N*¹-acetyl-*N*²-formyl-5-methoxykynuramin, 6-Hydroxymelatonin und *N*-acetylserotonin, zur Verwendung bei der Vorbeugung und Behandlung von vaginaler Verletzung wegen externer Strahlentherapie des Beckens und/oder wegen Brachystrahlentherapie des Beckens, wobei die Zusammensetzung 15 bis 30 Minuten vor externer Strahlentherapie des Beckens und/oder Brachystrahlentherapie des Beckens auf das vaginale Epithel topisch verabreicht wird.

2. Zusammensetzung zur Verwendung nach Anspruch 1, wobei die Zusammensetzung Schaum, Gel, Creme, Salbe oder ein Arzneistoff enthaltendes Pessar oder Suppositorium ist.

3. Zusammensetzung zur Verwendung nach einem der vorgehenden Ansprüche, wobei der pH-Wert der Zusammensetzung im Bereich von 3,8-4,5 liegt.

4. Zusammensetzung zur Verwendung nach einem der vorhergehenden Ansprüche, wobei die Zusammensetzung zur Verabreichung 1, 2, 3, 4, 5 oder 6 Mal pro Tag ist.

5. Zusammensetzung zur Verwendung nach einem der vorhergehenden Ansprüche, wobei die Zusammensetzung zur Verabreichung über einen Zeitraum von 3 Monaten oder mehr, wie beispielsweise 4 Monaten oder mehr, wie beispielsweise 5 Monaten oder mehr, wie beispielsweise 6 Monaten oder mehr, wie beispielsweise 12 Monaten, ist.

6. Zusammensetzung zur Verwendung nach einem der vorgehenden Ansprüche, wobei die Zusammensetzung in der einzelnen Standarddosis von Melatonin für Erwachsene von 1 mg bis 100 mg ist.

7. Zusammensetzung zur Verwendung nach einem der vorhergehenden Ansprüche, wobei die tägliche Dosis der Zusammensetzung vom Ein- bis Sechsfachen der Einzeldosen nach Anspruch 6 beträgt.

## Revendications

1. Composition comprenant de la mélatonine de formule (I) 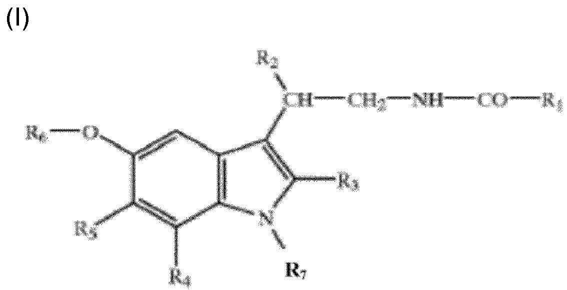
dans laquelle R₁ et R₆ représentent CH₃, et R₂, R₃, R₄, R₅ et R₇ représentent H, ou un dérivé d'antioxydant de celle-ci, dans laquelle
R₁ représente H, un groupe d'alkyle en C₁-C₄ linéaire ou ramifié, ou un groupe d'alkoxy en C₁-C₄,
R₂ représente H ou un groupe d'alkyle en C₁-C₄,
R₃ représente H, un groupe méthyle ou un atome d'halogène,
R₄ représente H ou un atome d'halogène,
R₅ représente H ou un atome d'halogène,
R₆ représente H, un groupe d'alkyle en C₁-C₄ linéaire ou ramifié,
R₇ représente H, un groupe d'alkyle en C₁-C₄ linéaire ou ramifié, un groupe - C(=O)-O-Rₐ u un groupe -C(=O)-N(H)-Rₐ, dans laquelle Rₐ un groupe d'alkyle en C₁-C₄ linéaire ou ramifié,
ou des métabolites antioxydants de celle-là de la formule *N*¹-acétyle-*N*²-formyl-5-methoxykynuramine, 6-hydroxymélatonine et *N*-acetylérotonine, à utiliser dans la prévention et le traitement des lésions vaginales dues à la radiothérapie du faisceau externe pelvien et / ou la brachyradiothérapie pelvienne, la composition étant administrée par voie topique à l'épithélium vaginal 15 minutes à 30 minutes avant un épisode de la radiothérapie du faisceau externe pelvien et / ou de la brachyradiothérapie pelvienne.

2. Composition pour l'usage selon la revendication 1, dans laquelle la composition est sous forme de mousse, gel, crème, onguent ou pessaire ou suppositoire médicamenteux.

3. Composition pour l'usage selon l'une quelconque des revendications précédentes, dans laquelle le pH de la composition est dans la plage de 3,8 et 4,5.

4. Composition pour l'usage selon l'une quelconque des revendications précédentes, dans laquelle la composition est destinée à une administration 1, 2, 3, 4, 5 ou 6 fois par jour.

5. Composition pour l'usage selon l'une quelconque des revendications précédentes, dans laquelle la composition est destinée à une administration sur une période allant jusqu'à 3 mois ou plus, telle que 4 mois ou plus, telle que 5 mois ou plus, telle que 6 mois ou plus, telle que comme 12 mois.

6. Composition selon l'une quelconque des revendications précédentes, dans laquelle la composition est dans la dose unique standard adulte de mélatonine de 1 mg à 100 mg.

7. Composition pour l'usage selon l'une quelconque des revendications précédentes, dans laquelle la dose quotidienne de composition est d'une à six fois la dose unique de la revendication 6.
